(19) Europäisches Patentamt
European Patent Office
Office européen des brevets

(11) **EP 4 018 019 B1**

(12) **EUROPÄISCHE PATENTSCHRIFT**

(45) Veröffentlichungstag und Bekanntmachung des Hinweises auf die Patenterteilung:
**21.12.2022 Patentblatt 2022/51**

(21) Anmeldenummer: **20743691.6**

(22) Anmeldetag: **21.07.2020**

(51) Internationale Patentklassifikation (IPC):
*G01N 1/32* (2006.01)    *G01N 33/2045* (2019.01)
*C30B 29/06* (2006.01)    *C30B 13/28* (2006.01)

(52) Gemeinsame Patentklassifikation (CPC):
**C30B 29/06; C30B 13/28; G01N 1/32; G01N 33/2045**

(86) Internationale Anmeldenummer:
**PCT/EP2020/070562**

(87) Internationale Veröffentlichungsnummer:
**WO 2022/017586 (27.01.2022 Gazette 2022/04)**

(54) **VERFAHREN ZUR BESTIMMUNG VON SPURENMETALLEN IN SILICIUM**

METHOD FOR DETERMINING TRACE METALS IN SILICON

PROCÉDÉ DE DÉTERMINATION DE MÉTAUX TRACES DANS DU SILICIUM

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**29.06.2022 Patentblatt 2022/26**

(73) Patentinhaber: **Wacker Chemie AG**
**81737 München (DE)**

(72) Erfinder:
• **ASCHL, Waltraud**
**84387 Julbach (DE)**

• **KAUTNICK, Theresa**
**84489 Burghausen (DE)**
• **STADLMAYR, Manuel**
**84478 Waldkraiburg (DE)**
• **STEINKRESS, Peter**
**5280 Braunau (AT)**

(74) Vertreter: **Belz, Ferdinand et al**
**Wacker Chemie AG**
**Hanns-Seidel-Platz 4**
**81737 München (DE)**

(56) Entgegenhaltungen:
**EP-A2- 0 349 117    DE-A1-102010 039 755**

EP 4 018 019 B1

**Beschreibung**

Die Erfindung betrifft ein Verfahren zur Bestimmung von metallischen Verunreinigungen in Silicium.

Polykristallines Silicium (Polysilicium) dient als Ausgangsmaterial bei der Herstellung von einkristallinem Silicium, beispielsweise mittels Tiegelziehen (Czochralski-Verfahren) oder mittels Zonenschmelzen (Floatzone-Verfahren). Das einkristalline Silicium kann in Scheiben (Wafer) zersägt und nach einer Vielzahl weiterer Bearbeitungsschritte in der Halbleiterindustrie zur Fertigung von elektronischen Bauelementen (z.B. Widerstände, Dioden, Bipolar- und MOS-Transistoren) verwendet werden. Diese Bearbeitungsschritte umfassen in der Regel ein gezieltes und lokal begrenztes Verunreinigen des einkristallinen Siliciums mit Dotierstoffen. Dazu zählen insbesondere Atome mit drei oder fünf Valenzelektronen, also beispielsweise die Elemente der 3. und 5. Hauptgruppe des Periodensystems. Voraussetzung für die Fertigung dieser Halbleiterbauelemente ist daher, dass das verwendete Silicium in höchster Reinheit (keine Fremddotierung) und als perfekter Einkristall vorliegt, da auch Korngrenzen und Gitterfehler zu unerwünschten Strompfaden führen können. Für die verschiedenen Anwendungen von Polysilicium gibt es hinsichtlich der Verunreinigungen vorgegebene Spezifikationen, die nicht überschritten werden dürfen.

Grundsätzlich können bereits Verunreinigungen (bspw. Bor, Phosphor und Arsen) im ppt-Bereich (parts per trillion, $10^{-12}$) die gewünschte Leistung von Siliciumhalbleitern verändern. Neben den Dotieratomen können insbesondere auch Spurenmetall- und Nichtmetallverunreinigungen zu Defekten beitragen. Zu nennen wären hier beispielsweise die Nichtmetalle Kohlenstoff, Chlor, und metallische Verunreinigungen mit Eisen, Chrom, Nickel oder Kupfer.

Um sicherzustellen, dass die Verunreinigungen niedriger sind als durch die Spezifikation vorgegeben, ist ein entsprechend empfindliches Analyseverfahren erforderlich. Ferner sind für eine routinemäßige Überwachung der Verunreinigungen praktische Gesichtspunkte wie Analysedauer und Reproduzierbarkeit wichtig.

Bei metallischen Verunreinigungen wird prinzipiell unterschieden zwischen Metallen, die auf der Oberfläche des Polysiliciums vorhanden sind, und Metallen, die sich in der Tiefe des Polysiliciums (Bulk) befinden. Im Folgenden geht es um ein Verfahren zur Bestimmung von metallischen Verunreinigungen im Silicium-Bulk.

Ein bekanntes Verfahren ist die Instrumentelle Neutronen-Aktivierungsanalyse (INAA). Dabei werden Polysiliciumproben nach einer Behandlung ihrer Oberfläche mit einer Reinigungsätze in einem Kernreaktor bestrahlt. Die metallischen Kontaminationen verwandeln sich dabei in radioaktive Isotope. Die beim Zerfall der radioaktiven Kerne entstehende elementspezifische Gamma-Strahlung wird gemessen und kann den Elementen zugeordnet werden.

Die INAA hat den Vorteil, dass sie für die meisten Metalle sehr niedrige Nachweisgrenzen besitzt. Diese liegen für Fe, Cr und Ni typischerweise bei kleiner gleich 50 pg/g [pptw] Silicium. Für andere metallische Verunreinigungen wie beispielsweise Ca oder Ti liegen die Nachweisgrenzen bei 500 bis 2000 pg/g. Nachteilig ist der hohe instrumentelle Aufwand, da eine Neutronenquelle (Kernreaktor oder Teilchenbeschleuniger) notwendig ist. Ein weiterer Nachteil ist das Erfordernis mehrerer zeitlich versetzter Messungen, da verschiedene Elemente unterschiedliche Zerfallszeiten aufweisen. Die Dauer einer INAA kann 2 bis 3 Monate betragen. Zwar ist die INAA nachweisstark, allerdings als prozessbegleitende Analysemethode (z.B. Routineanalysen zur Optimierung von Parametern), bei der kurze Messdauern wünschenswert sind, ungeeignet. Zudem ist diese Analyse sehr teuer.

Eine weitere Methode stellt das Lösen einer Siliciumprobe ohne vorherige Anreicherung der Verunreinigungen dar. Dabei wird die Probe in einer wässrigen Ätzlösung aus konzentrierter Salpetersäure ($HNO_3$) und Flusssäure (HF) komplett gelöst, die Matrix abgeraucht und die metallischen Spuren in einer verdünnten Säure wieder aufgenommen. Die Probe kann dann mit einem spurenanalytischen Verfahren untersucht werden, z.B. Atomabsorptionsspektrometrie (AAS), Massenspektrometrie mit induktiv gekoppeltem Plasma (ICP-MS), optische Emissionsspektrometrie mit induktiv gekoppeltem Plasma (ICP-OES).

Der Vorteil liegt in der kurzen Durchlaufzeit der Analyse von typischerweise 1 bis 2 Tagen. Da das Verfahren keinen Anreicherungsschritt beinhaltet, liegen die Nachweisgrenzen im Vergleich zur INAA üblicherweise um einen Faktor 100 höher.

Eine weitere Methode ist aus EP 0 349 117 A2 als "Freeze-Tip"-Verfahren bekannt. Dabei werden die metallischen Verunreinigungen in einem Siliciumstab im ersten Schritt mittels Zonenschmelzen (Zonenziehen) in der Schmelze des Stabs mitgeführt und angereichert. Am Ende des Zonenschmelzens wird die Energiezufuhr gestoppt und die Schmelze erstarrt. Das abgekühlte Stück mit den angereicherten Verunreinigungen wird vom entstandenen Einkristall getrennt und in einer wässrigen Säure vollständig gelöst. Diese Lösung kann anschließend mit einem der genannten spurenanalytischen Verfahren analysiert werden.

Ein Vorteil des "Freeze-Tip"-Verfahrens liegt in seiner für Routineanalysen tauglichen Durchlaufzeit, die typischerweise kleiner als eine Woche ist. Nachteilig ist, dass die Schmelze bzw. der Tip spontan erkaltet und sich daher ein undefinierter Kristallisationszustand bildet. Dies liegt insbesondere an der nur teilweise wirkenden Segregation und führt zu einer inhomogenen Verteilung der metallischen Kontaminationen im erstarrten Silicium. Ein weiterer Nachteil liegt im erforderlichen mechanischen Abtrennen des erkalteten Tips vom Einkristall. Beispielsweise werden dafür Diamantsägen, Diamant-Ritzer oder Zangen eingesetzt, wodurch es in der Regel zu metallischen Kontaminationen kommt, die vor dem Lösen beseitigt werden müssen. Dies geschieht durch ein oberflächliches Ätzen, wodurch allerdings zwangsläufig auch Teile des zu untersuchenden Tips entfernt werden und der eigentlichen Spurenmetallanalyse verloren gehen. Das Analyseergebnis wird dadurch verfälscht.

Die DE 10 2010 039 755 A1 beschreibt ebenfalls ein auf dem Zonenschmelzen beruhendes Verfahren, wobei im Unterschied zum "Freeze-Tip"-Verfahren zwischen dem entstandenen Einkristall und dem Siliciumstab durch spezielle Ziehtechnik ein tropfenförmiges, kristallines Probestück ("Freeze-Nub") am Ende des Einkristalls erstarrt. Ein Vorteil des Verfahrens ist, dass der Nub nicht mechanisch von Einkristall abgetrennt werden muss, wodurch Kontaminationen und der Probenmaterialverlust verringert werden. Nachteilig sind die geringeren Wiederfindungsraten in einer Größenordnung von 10-40 %. Der Grund dafür ist der große Anteil von sich in der Schmelze befindlichem Silicium, der beim Trennen am Probenstück zurückbleibt. Damit einher gehen höhere Nachweisgrenzen. Die Nachweisgrenze errechnet sich aus folgender Formel:

$$\mathrm{NWG} \; = \; \frac{3\sigma \, RLV \left(\frac{ng}{ml}\right) 100\%}{m_{Si} \; WFR},$$

wobei

NWG: Nachweisgrenze,
RLV: Rücklösevolumen,
WFR: Wiederfindungsrate,
$m_{Si}$: Masse umgeschmolzenes Silicium
$\sigma$: Standardabweichung der Blindwerte

Da die Wiederfindungsrate in der Formel im Nenner steht, wird eine niedrige Wiederfindungsrate zu einer hohen Nachweisgrenze. Darüber hinaus wird der Freeze-Nub komplett gelöst, was größere Mengen Säure erfordert. Auch dies hat höhere Nachweisgrenzen zur Folge, da die Säure Verunreinigungen enthält.

Die Aufgabe der Erfindung bestand darin, ein verbessertes Verfahren zur Bestimmung der Reinheit von Silicium bereitzustellen.

Die Aufgabe wird gelöst durch ein Verfahren zur Bestimmung von metallischen Verunreinigungen (Spurenmetallen und Spurenhalbmetallen) in Silicium, umfassend die Schritte:

a) Bereitstellen einer stabförmigen Siliciumprobe und eines stabförmigen Impfkristalls in einer Zonenschmelzvorrichtung;
b) Zonenschmelzen (Zonenziehen) unter Ausbildung eines Siliciumeinkristalls mit einem konischen Endbereich, wobei sich in einem Trennschritt eine tropfenförmige Schmelze am Ende des Siliciumeinkristalls formt;
c) Abkühlen der tropfenförmigen Schmelze unter Ausbildung eines erstarrten Siliciumtropfens (Pin-Nub);
d) Teilweises oder vollständiges Lösen des Siliciumtropfens in einer Säure;
e) Analysieren der in Schritt d) erhaltenen Lösung mit einer spurenanalytischen Methode.

Das Verfahren zeichnet sich dadurch aus, dass der Trennschritt im Verfahrensschritt b) die folgenden chronologischen Teilschritte umfasst:

- Rückschmelzen der Siliciumprobe zur Verringerung ihres Durchmessers, wobei für ein erstes Zeitintervall die Bewegungsrichtung der Siliciumprobe und des Impfkristalls gegenüber ihrer vorherigen Bewegungsrichtung zur Ausbildung des konischen Endbereichs umgekehrt wird;
- Bilden einer tropfenförmigen Schmelzzone, wobei für ein zweites Zeitintervall die Bewegung des Impfkristalls gestoppt und die Bewegungsrichtung der Siliciumprobe erneut umgekehrt wird;
- Trennen von Impfkristall und Siliciumprobe, wobei die Bewegungsrichtung der Siliciumprobe umgekehrt wird und diese für eine Dauer von 5 bis 20 s eine Bewegungsgeschwindigkeit von 150 bis 400 mm/min aufweist.

Das erfindungsgemäße Verfahren stellt insbesondere eine Weiterentwicklung und Verbesserung des in der DE 10 2010 039 755 A1 beschriebenen Verfahrens dar.

Bei dem Silicium kann es sich um monokristallines, multikristallines und polykristallines Silicium handeln. Vorzugsweise handelt es sich polykristallines Silicium, das insbesondere nach dem Siemens-Verfahren hergestellt wurde.

Das Zonenschmelzen (auch als Zonenziehen oder Floating-Zone (FZ)-Verfahren bezeichnet) in einer Zonenschmelzvorrichtung ist aus dem Stand der Technik bekannt. Insbesondere kann hier auf den Übersichtsartikel *Historical overview of silicon pulling development* (W. Zulehner - Materials Science and Engineering: B, 3 April 2000, p. 7-15, Elsevier) und die DE 10 2010 039 755 A1 verweisen werden.

Kurz zusammengefasst wird üblicherweise zunächst mittels Kernbohrung eine stabförmige Siliciumprobe (Bohrkern) entnommen. Diese stammt insbesondere aus einem gemäß dem Siemens-Verfahren hergestellten Polysiliciumstab. Die Siliciumprobe hat für gewöhnlich eine Länge von 3 bis 25 cm, bevorzugt von 5 bis 15 cm und einen Durchmesser von 10 bis 50 mm, bevorzugt von 15 bis 25 mm und besonders bevorzugt von 17 bis 21 mm.

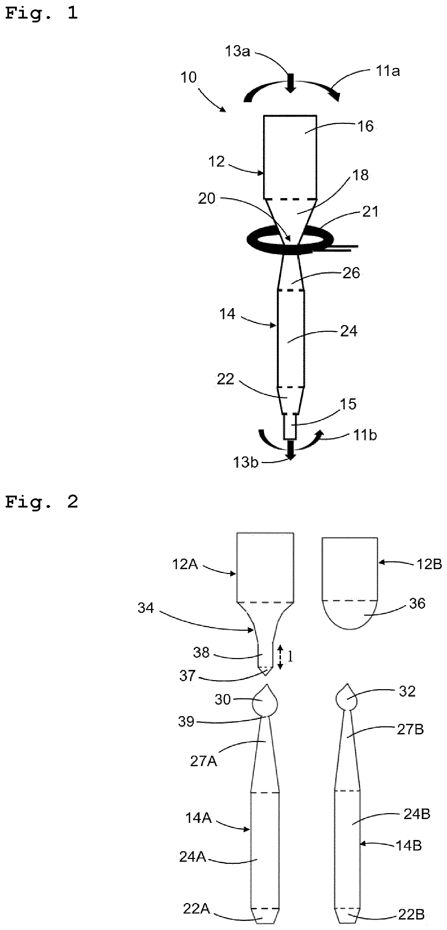

Die Siliciumprobe wird üblicherweise vor dem Einspannen in die Zonenschmelzvorrichtung gereinigt (Lösungsmittel- und/oder Säurebehandlung). Beim Zonenschmelzen wird die Siliciumprobe durch eine Induktionsspule (Induktionsheizung) an einem Ende in einer schmalen Zone aufgeschmolzen. Die aufgeschmolzene Zone (Schmelze) wird mit einem stabförmigen Siliciumeinkristall in Berührung gebracht und wächst unter Annahme seiner Kristallstruktur an ihm an. Diese Schmelzzone wird nun langsam durch die stabförmige Siliciumprobe bewegt. Mit anderen Worten wird die Bewegungsrichtung beider Stäbe derart gesteuert, dass sich die Schmelzzone (Induktionsspule) entlang der Siliciumprobe bewegt. Beide Stäbe haben währenddessen also dieselbe Bewegungsrichtung (vgl. Fig. 1). Durch unterschiedliche Bewegungsgeschwindigkeiten der Siliciumprobe und des heranwachsenden Einkristalls wird ein konischer Endbereich des Einkristalls erhalten. Damit die Zone gleichmäßig aufschmilzt, rotieren beide Stäbe.

Der entstehende Einkristall hat bezogen auf seinen zylindrischen Bereich in Abhängigkeit vom Durchmesser der Siliciumprobe einen Durchmesser von typischerweise 5 bis 50 mm, bevorzugt von 8 bis 25 mm, besonders bevorzugt von 10 bis 15 mm. Die Kristalllänge ist generell vom Durchmesser und von der Länge der Siliciumprobe abhängig.

Es hat sich gezeigt, dass sich die Wiederfindungsrate im Vergleich zum Freeze-Nub-Verfahren gemäß DE 10 2010 039 755 A1 steigert, wenn vor dem Bilden der tropfenförmigen Schmelzzone der Durchmesser der Siliciumprobe in ihrem Endbereich durch Rückschmelzen reduziert wird.

Beim Freeze-Nub-Verfahren existiert im Zeitpunkt des Trennens eine tropfenförmige Schmelzzone zwischen der Siliciumprobe und dem Einkristall. Beim Trennen rekristallisiert der Hauptteil dieser tropfenförmigen Schmelzzone (ca. 50 bis 80 %) an der Siliciumprobe und nur 20 bis 50 % am Einkristall in der Form des Nubs. Der Anteil, der an der Siliciumprobe rekristallisiert, geht der Analyse verloren. Um diesen Anteil zu reduzieren, wird beim erfindungsgemäßen Verfahren der Durchmesser des Endbereichs der Siliciumprobe reduziert. Mit anderen Worten wird der Durchmesser der Fläche der Siliciumprobe, die mit der tropfenförmigen Schmelzzone kontakt hat, reduziert. Um dies zu erreichen, wird die Bewegungsrichtung des Einkristalls und der Siliciumprobe im Vergleich zum oben beschriebenen üblichen Verlauf des Zonenschmelzens umgekehrt. Es wird also für ein Zeitintervall die Siliciumprobe von der tropfenförmigen Schmelzzone bzw. der Induktionsspule wegbewegt, was zur Reduzierung des Durchmessers im Endbereich der Siliciumprobe führt.

Überraschenderweise hat sich herausgestellt, dass sich dadurch die metallischen Verunreinigungen in einem Anteil von bis zu 95 % in dem erstarrten Siliciumtropfen (Nub) wiederfinden. Im Vergleich dazu hat das Freeze-Nub-Verfahren nur eine durchschnittliche Wiederfindungsrate von ca. 40 %.

Vorzugsweise weist nach dem Rückschmelzen die Siliciumprobe in einem Endbereich der Länge 1 einen Durchmesser auf, der kleiner oder gleich dem Durchmesser ist, den der Einkristall an seiner Kontaktfläche mit der Schmelze aufweist.

Der Durchmesser der Kontaktfläche des Einkristalls mit der Schmelze beträgt bevorzugt 3 bis 8 mm, besonders bevorzugt 4 bis 6 mm. Der Durchmesser der Siliciumprobe in ihrem Endbereich der Länge l beträgt vorzugsweise 2 bis 8 mm, besonders bevorzugt 3 bis 6 mm. Insbesondere beträgt sowohl der Durchmesser der Kontaktfläche des Einkristalls als auch der des Endbereichs der Länge l ca. 5 mm ($\pm$ 0,5 mm).

Vorzugsweise entspricht die Länge l des Endbereichs der Siliciumprobe ihrem einfachen bis dreifachen Durchmesser.

Beim Rückschmelzen wird die Siliciumprobe bevorzugt mit einer höheren Geschwindigkeit bewegt als der Einkristall. Vorzugsweise entspricht die Bewegungsgeschwindigkeit des Einkristalls etwa der halben Geschwindigkeit der Siliciumprobe.

Die Bewegungsgeschwindigkeit der Siliciumprobe kann 5 bis 15 mm/min, bevorzugt 7 bis 13 mm/min, besonders bevorzugt 9 bis 11 mm/min, betragen.

Die Bewegungsgeschwindigkeit des Einkristalls kann 2 bis 10 mm/min, bevorzugt 3 bis 8 mm/min, besonders bevorzugt 4 bis 6 mm/min, betragen.

Das erste Zeitintervall beim Rückschmelzen, in dem eine Umkehr der Bewegungsrichtung erfolgt, beträgt vorzugsweise 30 bis 300 s, besonders bevorzugt 90 bis 240 s, insbesondere 90 bis 120 s.

Im Anschluss an das Rückschmelzen wird die tropfenförmigen Schmelzzone ausgebildet. Dies geschieht durch ein Stoppen der Bewegung des Impfkristalls und eine zeitgleiche erneute Umkehr der Bewegungsrichtung der Siliciumprobe. Die Bildung der tropfenförmigen Schmelzzone findet in einem zweiten Zeitintervall statt, das vorzugsweise 1 bis 4 s, bevorzugt 2 bis 3 s, andauert. Die Bewegungsgeschwindigkeit der Siliciumprobe kann dabei 1 bis 5 mm/min, bevorzugt 2 bis 4 mm/min, betragen.

Die Trennung von Impfkristall und Siliciumprobe erfolgt langsam während einer Dauer von 5 bis 20 s mit einer Bewegungsgeschwindigkeit der Siliciumprobe von 150 bis 400 mm/min, vorzugsweise 250 bis 350 mm/min, wobei die Bewegungsrichtung im Vergleich zur Ausbildung der tropfenförmigen Schmelzzone wieder umgekehrt ist und der Einkristall nach wie vor unbewegt (abgesehen von der Rotation) ist. Die Induktionsheizung wird vorzugsweise zu Beginn der Trennung ausgeschaltet. Nach der Trennung beginnt die tropfenförmigen Schmelzzone abzukühlen.

Es hat sich gezeigt, dass die langsame Trennung dazu führt, dass sich in einer äußeren Schicht des erstarrenden Siliciumtropfens (Pin-Nubs) bereits alle enthaltenen Verunreinigungen ansammeln und somit auf ein vollständiges Auflösen des Pin-Nubs verzichtet werden kann.

Eine mögliche Erklärung für diesen Effekt der entdeckten Anreicherung der metallischen Kontaminationen im äußeren Randbereich könnte ein Gettereffekt sein (vgl. W. Zulehner - Materials Science and Engineering: B, 3 April 2000, p. 7-15, Elsevier). Dabei präzipitieren im Festkörper bei hohen Temperaturen gelöste metallische Verunreinigungen beim Abkühlen des Festkörpers an Defektstellen bevorzugt aus und reichern sich somit an diesen Defektstellen an. Die Oberfläche des Pin-Nubs, bei dem das Kristallgitter natürlicherweise endet und somit an der Oberfläche offene Bindungen trägt, ist eine solche Defektstelle. Die Diffusionsgeschwindigkeit der metallischen Verunreinigungen ist generell eine Funktion der Temperatur und bei hohen Temperaturen ist die Diffusion schneller als bei niedrigen Temperaturen (vgl. K. Graff, Metal Impurities in Silicon-Device Fabrication, ISBN 978-3-642-62965-5). Deshalb könnte eine langsame Abkühlphase des Pin-Nubs das Gettern verstärken (langsames Wegbewegen des noch heißen Endes der Siliciumprobe) und es erfolgt eine Diffusion der metallischen Verunreinigungen an die Oberfläche als eine natürliche Defektstelle des Siliciums.

Zum Abkühlen in Schritt c) kann ferner die Bewegung der Siliciumprobe gestoppt und zeitgleich der Einkristall mit einer Bewegungsgeschwindigkeit von 150 bis 400 mm/min, bevorzugt 250 bis 350 mm/min, in seine ursprüngliche Bewegungsrichtung (übliche Bewegungsrichtung des Zonenschmelzens) versetzt werden. Dies kann zu einer zusätzlichen Verstärkung des Gettereffekts führen.

Generell kann das Abkühlen in Schritt c) allerdings auch durch bloßes Ruhenlassen, ggf. unter Ausschalten der Rotation, erfolgen.

Der erstarrte Siliciumtropfen (Pin-Nub) hat üblicherweise einen Durchmesser von 3 bis 10 mm, bevorzugt 6 bis 8 mm. Der Pin-Nub entspricht für gewöhnlich weniger als 1 Gew.-% der aufgeschmolzenen Siliciumprobe. Typischerweise liegt das Gewicht eines Pin-Nubs (ohne den anhaftenden Einkristall) bei 3 bis 5 g.

Wie beim Freeze-Nub-Verfahren muss der Pin-Nub zum Lösen nicht vom Einkristall abgetrennt werden. Er wird vorzugsweise nach dem Abkühlen zusammen mit dem Einkristall in einen staubreien Beutel überführt. Somit kann er berührungs- und damit kontaminationsfrei in eine Halteapparatur geklemmt und einem nasschemischen Aufschlussverfahren zugeführt werden. Dieses findet vorzugsweise unter Reinraumbedingungen statt, wobei der Beutel ggf. dann im Reinraum entfernt wird.

Da sich die metallischen Verunreinigungen hauptsächlich in einer äußeren Schicht des Pin-Nubs befinden, ist es mit besonderem Vorteil nicht erforderlich, den Pin-Nub komplett aufzulösen. Vielmehr genügt ein Anätzen des Pin-Nubs durch ein zeitweises Eintauchen in eine Ätzlösung (Säure), wodurch sich je nach Dauer des Eintauchens nur eine mehr oder weniger dicke Außenschicht auflöst. Es hat sich gezeigt, dass bei einem etwa 3 g schweren Pin-Nub bereits in einem Siliciumabtrag von 0,1 bis 0,2 g alle metallischen Verunreinigungen enthalten sind.

Gegebenenfalls kann vor dem Eintauchen des Pin-Nubs in die Säure eine abtragsfreie Oberflächenreinigung, bspw. mit verdünnter Salpetersäure, erfolgen. Allerdings hat sich gezeigt, dass durch diese Prozedur eine Kontamination der Probe wahrscheinlich ist. Ein unmittelbares Überführen in einen staubfreien Beutel ist bevorzugt.

Vorzugsweise erfolgt das Eintauchen des Pin-Nubs in die Säure für eine Dauer von 3 bis 15 min, besonders bevorzugt von 5 bis 10 min, insbesondere von etwa 6 min. Im Vergleich dazu beträgt die Ätzdauer zum kompletten Lösen eines Freeze-Nubs durchschnittlich 2 Stunden. Eine verkürzte Ätzdauer bedeutet generell bessere Blindwerte und somit verbesserte Nachweisgrenzen. Je länger die Proben und die Säure der Umgebung ausgesetzt sind, umso größer ist die Wahrscheinlichkeit, dass sie kontaminiert werden.

Die Säure bzw. Ätzlösung umfasst vorzugsweise eine Mischung aus konzentrierter Salpetersäure (50-80 Gew.-%) und Flusssäure (20-50 Gew.-%) im Verhältnis 4:1 bis 3:1, bevorzugt, 2:1 bis 1:1. Die Ätzlösung kann bei Raumtemperatur oder erwärmt (z.B. 60°C) verwendet werden. Bevorzugt wird auf eine Erwärmung verzichtet. Das Überführen auf eine Heizvorrichtung stellt eine weitere Kontaminationsquelle dar.

Die Menge an Säure wird generell so gewählt, dass diese gerade ausreichend ist, um eine äußere Schicht des Pin-Nubs zu lösen. Üblicherweise wird eine Gesamtmenge an Ätzlösung von 5-10 ml eingesetzt. Um die Nachweisgrenzen der enthaltenen Verunreinigungen vom Chemikalieneinfluss zu entkoppeln, sollte grundsätzlich die Menge an verwendeten Chemikalien minimiert werden. Hier ist das Pin-Nub-Verfahren gegenüber dem Freeze-Nub-Verfahren im Vorteil.

Vorzugsweise erfolgt das teilweise Auflösen des Pin-Nubs folgendermaßen:

1. Bereitstellung der Ätzlösung,
2. Eintauchen des Pin-Nubs in die Ätzlösung für 3-15 min,
3. Abspülen des aus der Ätzlösung entfernten Pin-Nubs mit frischer Ätzlösung,

4. Einengen der Ätzlösung durch Erhitzen auf 100 bis 350°C.

Abhängig von der im Anschluss verwendeten spurenanalytischen Messmethode werden die nach dem Einengen verbliebenen metallischen Spuren in verdünnter Salpetersäure (0.5 bis 5 Gew.-%) und/oder verdünnter Flusssäure unter Erhalt einer Messlösung rückgelöst. Durch den verringerten Ätzabtrag und der dadurch verringerten Siliciummatrix (gelöstes Silicium ist als Hexafluorokieselsäure in der Ätzlösung enthalten) in der Messlösung konnte das Rücklösevolumen im Vergleich zum Freeze-Nub-Verfahren um durchschnittlich die Hälfte (typischerweise von 3 auf 1,5 ml) reduziert werden, wodurch sich die Konzentration der metallischen Verunreinigungen erhöht. Die Messung kann somit mit höheren Konzentrationen durchgeführt werden, was zu stabileren Messergebnissen führt, da das Signal im Verhältnis zum Rauschen erhöht wird.

Zur Analyse in Schritt e) können übliche Massenspektrometer wie ICP-MS eingesetzt werden. Weitere bevorzugte Messmethoden sind Atomabsorptionsspektrometrie mit elektrothermischer Aufheizung (*engl.:* Graphite Furnace Atomic Absorption Spectrometry, GFAAS) und Totalreflexions-Röntgenfluoreszenzanalyse (TRFA).

| | |
|---|---|
| **Figur 1** | zeigt das Zonenschmelzen vor dem Trennschritt gemäß Stand der Technik. |
| **Figur 2** | zeigt einen Vergleich zwischen Pin-Nub- und Freeze-Nub-Verfahren. |
| **Figur 3** | zeigt einen Pin-Nub an einem Einkristall. |
| **Figur 4** | zeigt einen Pin-Nub in einem Säurebad. |
| **Figur 5** | zeigt die Konzentration von Eisen in einer Stufenätze. |
| **Figur 6** | zeigt die Konzentration von Kupfer in einer Stufenätze. |

Fig. 1 zeigt schematisch eine Zonenschmelzvorrichtung 10 mit einer Siliciumprobe 12 und einem Einkristall 14, die sich kurz vor dem Trennschritt befinden. Die Siliciumprobe 12 und der Einkristall 14 sind jeweils in eine rotierende Welle eingespannt, die aus Gründen der Übersichtlichkeit nicht dargestellt ist. Die Rotationsrichtung wird durch die Pfeile 11a, 11b, die Bewegungsrichtung durch die Pfeile 13a, 13b angedeutet. Die Siliciumprobe 12 und der Einkristall 14 sind über eine Schmelzzone 20 miteinander verbunden, die durch eine Induktionsspule 21 geheizt wird. Bei der Siliciumprobe 12 handelt es sich um eine stabförmige, Polysiliciumprobe mit einem zylindrischen Bereich 16 und einem in der Schmelzzone 20 konischen Bereich 18. Die Polysiliciumprobe hatte ursprünglich eine Länge von etwa 15 cm. Der Durchmesser des Bereichs 16 beträgt etwa 20 mm. Der an einem Impf-Einkristall 15 rekristallisierte Einkristall 14 besteht aus einem Anfangskonus 22, einem zylindrischen Abschnitt 24 und einem in der Schmelzzone 20 konischen Abschnitt 26. Die Länge des Einkristalls 14 beträgt ca. 110 mm und der Durchmesser im zylindrischen Abschnitt 24 etwa 14 mm.

Fig. 2 zeigt einen Pin-Nub 30 an einem konischen Endbereich 27A eines Einkristalls 14A, der gemäß dem erfindungsgemäßen Verfahren hergestellt wurde. Im Vergleich dazu ist ein Freeze-Nub 32 (gemäß DE 10 2010 039 755 A1) an einem konischen Endbereich 27B eines Einkristalls 14B dargestellt. Die Endbereiche 27A, 27B können eine Länge von 3 bis 6 cm aufweisen. Die Nummerierung von bereits in der Fig.1 bezeichneten Elementen wird beibehalten, wobei Elemente aus dem Pin-Nub-Verfahren mit einem "A" und Elemente aus dem Freeze-Nub-Verfahren mit einem "B" unterschieden werden. Die entsprechenden Siliciumproben 12A und 12B mit ihren aus den Verfahren resultierenden Endbereichen 34 und 36 sind ebenfalls dargestellt. Die Form der Einkristalle 24A, 24B unterscheidet sich nur unwesentlich. Der Durchmesser der zylindrischen Abschnitte 24A, 24B unterscheidet sich nicht notwendigerweise.

Der wesentliche sichtbare Unterschied nach der Durchführung beider Verfahren liegt in den Endbereichen 34, 36 der Einkristalle 12A, 12B. Bei Durchführung des Freeze-Nub-Verfahrens bildet der sich beim Trennen in der Schmelze befindliche Teil der Siliciumprobe 12B einen Rundkopfartigen Endbereich 36. Dieser Endbereich 36 entsprich in der Regel 50-80 % der Schmelze. Es verbleiben also nur 20-50 % in Form der Freeze-Nubs 32 am konischen Endbereich 27B des Einkristalls 24B. Im Gegensatz dazu bildet sich durch das Rückschmelzen beim Pin-Nub-Verfahren ein Endbereich 34 mit einem Teilstück 38 mit einer Länge l heraus, das vom Durchmesser maximal dem Durchmesser einer Kontaktfläche 39 zwischen dem konischen Endbereich 27A und Pin-Nub 30 entspricht. Auf diese Weise wird der beim Trennen in der Schmelze befindliche Teil der Siliciumprobe 12A auf einen kleinen Anteil 37 reduziert. Üblicherweise entspricht dieser Anteil 37 nur 5-10% der Schmelze beim Trennvorgang. Es gehen der Analyse für gewöhnlich also nur 5-10 % Siliciumschmelze verloren.

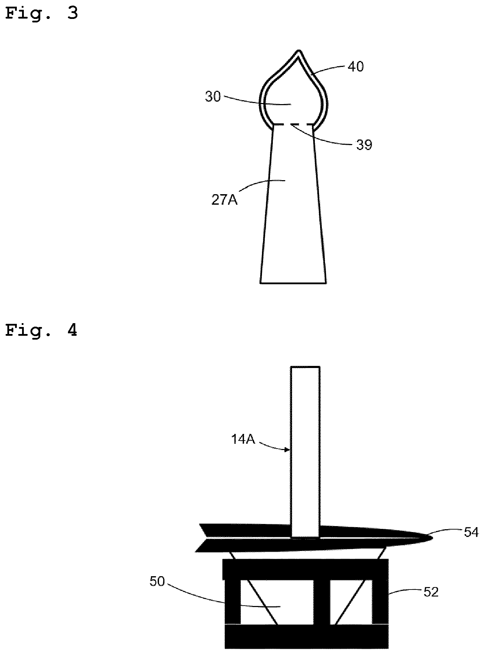

Fig. 3 zeigt einen Pin-Nub 30, der sich nach dem Abkühlen am konisch zulaufenden Endbereich 27A des Einkristalls 14A (vgl. Fig. 2) gebildet hat. Eine markierte Außenschicht 40 soll in etwa den Ätzabtrag verdeutlichen, der beim teilweisen Lösen des Siliciumtropfens in einer Säure für die nachfolgende Analyse aufbereitet wird. In dieser Außenschicht 40 sind die metallischen Verunreinigungen enthalten. Der Durchmesser der Kontaktfläche 39 mit dem Pin-Nub 30 beträgt etwa 5 mm. Im Wesentlichen entspricht dieser Durchmesser auch dem Durchmesser der Kontaktfläche des Einkristalls 14A in seinem konischen Endbereich 27A mit der Schmelze im Zeitpunkt des Trennens.

Fig. 4 zeigt ein konisches, mit Säure gefülltes Gefäß 50 in einer Halterung 52. Der Einkristall 14A mit dem Pin-Nub ist in das Gefäß 50 eingetaucht, wobei er durch eine Halteklammer 54 fixiert ist. Durch das konisch zulaufende Gefäß kann die zum teilweisen Lösen verwendetet Säuremenge reduziert werden.

**Beispiel 1**

Aus einem mit dem Czochralski-Verfahren gezogenem Siliciumstab, der mit einer bekannten Kontamination der Metalle Fe, Cr, Ni, Cu, Zn, Sn verunreinigt ist, wurden 6 Bohrkerne (Siliciumproben) entnommen, die alle einen Durchmesser von 22 mm aufwiesen und eine Länge von ca. 8 cm. Die Proben wurden mit einer Reinigungsätze für etwa 15 min lang in einem Säuregemisch aus HF (45%) und $HNO_3$ (65%) im Verhältnis 1:6 geätzt und mit Reinstwasser gespült. Die Präparation fand dazu unter Reinraumbedingungen (Klasse 10) statt.

Die Siliciumproben wurden jeweils in eine FZ-Vorrichtung eingebaut (obere Ziehwelle: Siliciumprobe; untere Ziehwelle: Impfling). Als Impfling diente ein Siliciumeinkristall. Der Zonenschmelzprozess ist in der Tabelle 1 aufgeführt, wobei auch auf die Figuren Bezug genommen wird. Bewegungsgeschwindigkeiten v, die mit einem negativen Vorzeichen versehen sind, deuten die übliche Ziehrichtung an, wenn die Schmelzzone durch die Siliciumprobe bewegt wird. Auf Fig. 1 und 2 bezogen bedeutet eine negative Bewegungsgeschwindigkeit, eine Bewegung des Einkristalls 14A und/oder der Siliciumprobe 12A im Verhältnis zur Induktionsspule 21 nach unten. Ein positives Vorzeichen deutet eine Bewegung nach oben an.

Tabelle 1

| Schritt | Pin-Nub-Verfahren |
|---|---|
| Vorheizen | • Mit Hilfe eines Vorheizers wird die Si-Probe 12A aufgeheizt<br>• Leistung (*P*) Generator: 36 kW (70% Leistung) |
| Einkoppeln | • Induktionsleistung koppelt ein<br>• Schmelztropfen an der Si-Probe 12A wird erzeugt |
| Ansetzen | • Schmelztropfen wird mit dem Impfling auf Höhe der Induktionsspule 21 verbunden (die beiden Ziehwellen werden manuell aufeinander zu bewegt) |
| Bildung Anfangskonus 22 | • Parameter für Durchmesser des zylindr. Absch. 24 von 14 mm:<br>*P* = 14 kW (40 % Leistung)<br>i *v* Ziehwelle oben: - 2,4 mm/min<br>*v* Ziehwelle unten: - 5,8 mm/min<br>Ziehlänge (Weg Ziehwelle unten): 5 mm |
| Zylindrischer Abschnitt 24A | *P* = 14,2 kW (40,5% Leistung)<br>*v* Ziehwelle oben: - 2,4 mm/min<br>*v* Ziehwelle unten: - 6,0 mm/min<br>Länge: (Weg Ziehwelle unten): 70 mm<br>Durchmesser: 14 mm |
| Übergang Bereich 27A | • Schritt 1:<br>*P* Generator: 13,7 kW (39,0 % Leistung)<br>*v* Ziehwelle oben: -1,8 mm/min<br>*v* Ziehwelle unten: - 10,0 mm/min<br>Ziehlänge (Weg Ziehwelle unten): 10 mm<br>• Schritt 2:<br>*P* = 13,5 kW (38,5 % Leistung)<br>*v* Ziehwelle oben: - 1,2 mm/min<br>*v* Ziehwelle unten: - 10,0 mm/min<br>Ziehlänge (unten): 10 mm |
| Bereich 27A | *P* = 13,3 kW (38 % Leistung)<br>*v* Ziehwelle oben: -1,0 mm/min<br>*v* Ziehwelle unten: - 14,5 mm/min<br>Ziehlänge (Weg Ziehwelle unten): 22 mm<br>Durchmesser Kontaktfläche 39: 5 - 6 mm |

(fortgesetzt)

| Schritt | Pin-Nub-Verfahren |
|---|---|
| Rückschmelzen | $P$ = 13,3 kW (38 % Leistung)<br>$v$ Ziehwelle oben: + 10 mm/min<br>$v$ Ziehwelle unten: + 6 mm/min<br>Weg Ziehwelle unten: 8 mm<br>Durchmesser Teilstück 38: 5 - 6 mm |
| Bilden Pin-Nub 30 | $P$ = 13,7 kW (39 % Leistung)<br>$v$ Ziehwelle oben: - 2,0 mm/min<br>$v$ Ziehwelle unten: 0 mm/min<br>Ziehlänqe (Weg Ziehwelle unten): 0 mm |
| Trennen | $P$ = 0 kW<br>$v$ Ziehwelle oben: + 350 mm/min<br>$v$ Ziehwelle unten: 0 mm/min<br>Weg Ziehwelle oben: 40 mm<br>Weq Ziehwelle unten: 0 mm |
| Abkühlen | Schritt 2:<br>$P$ = 0 kW<br>$v$ Ziehwelle oben: 0 mm/min<br>$v$ Ziehwelle unten: -350 mm/min<br>Weg Ziehwelle oben: 0 mm<br>Weg Ziehwelle unten: 300 mm |

Die Umdrehung des Einkristalls 14A betrug typischerweise von 15 bis 30 U/min und die der Siliciumprobe typischerweise 12A von 3 bis 10 U/min bei entgegengesetzter Drehrichtung (vgl. Fig. 1, Pfeile 11A, 11B). Nach Trennen und Abkühlen betrug der größte Durchmesser des Pin-Nubs ca. 8 mm.

Nach dem Ausbau wurde der Pin-Nub 30 mit einer Halteklammer 54 in ein mit einer Säure gefülltes konisches Gefäß 50 aus Perfluoralkoxylalkan (PFA) unter Reinraum-bedingungen (Klasse 10) überführt und teilweise gelöst (angeätzt). Die Säure bestand aus einem Gemisch aus $HNO_3$ (69 Gew.-%) und HF (40 Gew.- %) im Verhältnis 1:1. Das Gefäß 50 war mit ca. 6 ml Säure befüllt. Die Säure wirkte 6 min auf den vollständig eingetauchten Pin-Nub ein. Anschließend wurde der Pin-Nub mit 1 ml frischer Säure abgespült. Die erhaltene Ätzlösung wurde dann für ca. 30 min bei einer Temperatur von 250°C eingeengt. Der erhaltene Rest wurde mit einem Gemisch aus 25 µl HF (40 Gew.-%), 25 µl $HNO_3$ (65 Gew.-%) und 1450 ml Reinstwasser gelöst und eine Messlösung erhalten. Diese Behandlung wurde mit jedem der sechs erhaltenen Pin-Nubs durchgeführt.

Beim Messen mittels ICP-MS wurden die Elemente Fe, Cr, Ni, Na, K, Sn, Zn, Al, Cu, Mo, La, Cs, Ce, Te, Sc, Se, Ti, Ta, Ge, W, Mg, Ag, Li, V, Mn, Zr, Pb, Y, Sr, Ba, Bi, Cd, Sn, As, Ru, Rb, U, Ga, In, Ca und Co bestimmt. Zusätzlich wurden Blindwerte erzeugt. Dazu wurden 4 konische Gefäße mit Säure befüllt und analog ohne Proben präpariert. Die bei diesen Blindwerten gemessenen Gehalte wurden gemittelt und jeweils von den gemessenen Gehalten der Proben subtrahiert. Anschließend wurde der errechnete Wert auf das Gesamtgewicht des Einkristalls bezogen.

Die zeigt signifikant höhere Wiederfindungsraten (WFR) anhand ausgewählter Metalle im Vergleich zum Freeze-Nub-Verfahren. Es handelt es sich um Durchschnittswerte aus allen sechs Siliciumproben.

Tabelle 2

| WFR in % | Fe | Cr | Ni | Cu | Zn | Sn |
|---|---|---|---|---|---|---|
| Freeze-Nub | 37 | 40 | 54 | 38 | 37 | 41 |
| Pin-Nub | 94 | 95 | 95 | 96 | 94 | 94 |

Die Tabelle 3 zeigt erniedrigte Nachweisegrenzen im Vergleich zum Freeze-Nub-Verfahren.

EP 4 018 019 B1

Tabelle 3

| NWG (pg/g) | Fe | Cr | Ni | Cu | Zn | Na |
|---|---|---|---|---|---|---|
| Freeze-Nub | ≤ 20 | ≤ 20 | ≤ 20 | ≤ 10 | ≤ 10 | ≤ 20 |
| Pin-Nub | ≤ 2 | ≤ 1 | ≤ 1 | ≤ 1 | ≤ 1 | ≤ 1 |

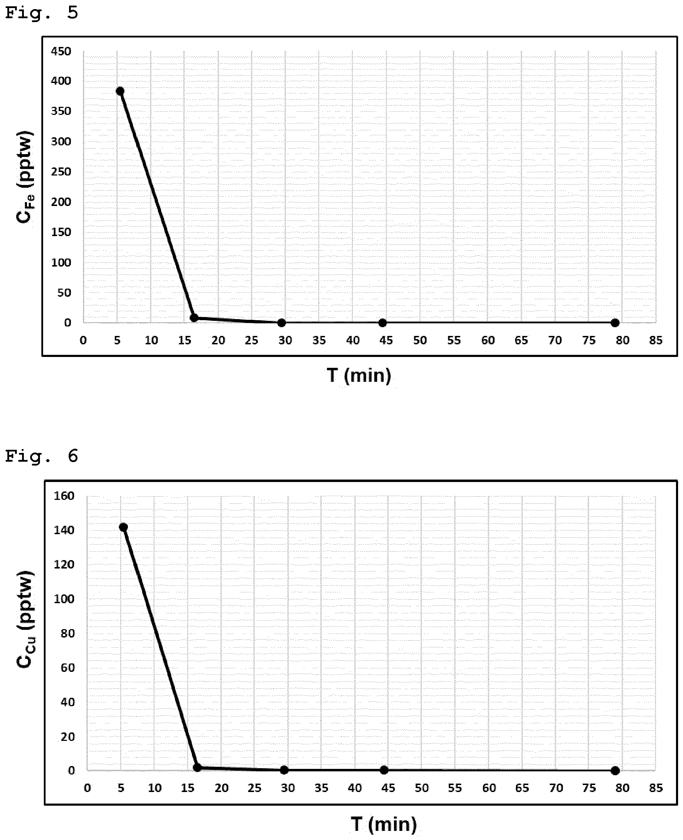

Die Fig. 5 und 6 zeigen den Konzentrationsverlauf von Eisen ($c_{Fe}$, Fig. 5) und Kupfer ($c_{Cu}$, Fig. 6) in Messlösungen, die nach 5 min, 16 min, 29 min, 44 min und 79 min Anätzen eines Pin-Nubs erhalten wurden (Stufenätze: der Pin-Nub wurde nach jeder Stufe wieder in frische Ätzlösung verbracht). Entsprechende Kurven wurden für Chrom und Nickel angefertigt, wobei jedes Experiment einmal wiederholt wurde (jeweils Probe 1 und 2). Die Ergebnisse sind in der Tabelle 4 dargestellt.

Tabelle 4

| Ätzabtrag pro Stufe in % | | | | |
|---|---|---|---|---|
| | Fe | Cr | Ni | Cu |
| 1. Ätzung Probe 1 | 98 | 96 | 98 | 98 |
| 2. Ätzung Probe 1 | 2 | 3 | 2 | 2 |
| 3. Ätzung Probe 1 | 0 | 0 | 1 | 0 |
| 4. Ätzung Probe 1 | 0 | 0 | 0 | 0 |
| 5. Ätzung Probe 1 | 0 | 0 | 0 | 0 |
| 1. Ätzung Probe 2 | 98 | 97 | 98 | 98 |
| 2. Ätzung Probe 2 | 2 | 2 | 1 | 1 |
| 3. Ätzung Probe 2 | 0 | 1 | 0 | 0 |
| 4. Ätzung Probe 2 | 0 | 0 | 0 | 0 |
| 5. Ätzung Probe 2 | 0 | 0 | 0 | 0 |

Bereits nach 5 min Ätzzeit und einem Abtrag von nur 0,06 g waren die metallischen Verunreinigungen in allen Fällen zu mindestens 96 % abgeätzt. Dieses Ergebnis zeigt, dass schon durch eine Reinigung eines Freeze-Tips (vgl. EP 0 349 117 A2) mit einer Reinigungsätze ein Teil der metallischen Verunreinigung der Analyse verloren geht.

**Beispiel 2**

Es wurden verschiedene Parameter beim Zonenschmelzen getestet:
Es wurden 7 Siliciumproben (Bohrkerne) mit 19 und 22 mm verwendet. Durch Anpassen der entsprechenden Parameter beim Zonenschmelzen wurden verschiedene Durchmesser (D) am Einkristall und am Endbereich der Siliciumproben erhalten. Außerdem wurden verschiedene Weglängen der unteren Ziehwelle beim Rückschmelzen getestet und verschiedene Pin-Nub-Größen erhalten. Zudem wurden Proben mit verschiedenem Gewicht des Einkristalls gezogen und getestet. Die Ergebnisse sind in der Tabelle 5 zusammengefasst.

Tabelle 5

| # | Einkristall (D) | Probenstück (D) | Umgeschmolzenes Silicium | Länge konischer Bereich | Länge Zurückschmelzen | (D) Probenende beim Trennen | Nub-Größe | WFR (Durchschnitte Fe, Cr, Ni, Cu) |
|---|---|---|---|---|---|---|---|---|
| | mm | mm | g | mm | mm | mm | g | % |
| 1 | 12 | 22 | 30 | 30 | 8 | 4 | 0,4 | 97 |
| 2 | 12 | 19 | 20 | 30 | 8 | 4 | 0,3 | 94 |
| 3 | 14 | 22 | 30 | 35 | 8 | 4 | 0,5 | 95 |
| 4 | 15 | 22 | 20 | 50 | 8 | 3 | 0,4 | 97 |
| 5 | 10 | 22 | 40 | 20 | 8 | 3 | 0,4 | 96 |
| 6 | 14 | 22 | 50 | 30 | 5 | 5 | 0,4 | 94 |
| 7 | 10 | 22 | 15 | 30 | 15 | 8 | 0,4 | 90 |

Die Wiederfindungsrate lag immer zwischen 90 und 97 %.

**Patentansprüche**

1. Verfahren zur Bestimmung von metallischen Verunreinigungen in Silicium, umfassend:

   a) Bereitstellen einer stabförmigen Siliciumprobe und eines stabförmigen Impfkristalls in einer Zonenschmelzvorrichtung;
   b) Zonenschmelzen unter Ausbildung eines Siliciumeinkristalls mit einem konischen Endbereich, wobei sich in einem Trennschritt eine tropfenförmige Schmelze am Ende des Siliciumeinkristalls formt;
   c) Abkühlen der tropfenförmigen Schmelze unter Ausbildung eines erstarrten Siliciumtropfens;
   d) Teilweises oder vollständiges Lösen des Siliciumtropfens in einer Säure;
   e) Analysieren der in Schritt d) erhaltenen Lösung mit einer spurenanalytischen Methode,

   **dadurch gekennzeichnet, dass** der Trennschritt die folgenden Teilschritte umfasst

   - Rückschmelzen der Siliciumprobe zur Verringerung ihres Durchmessers, wobei für ein erstes Zeitintervall die Bewegungsrichtung der Siliciumprobe und des Impfkristalls gegenüber ihrer vorherigen Bewegungsrichtung zur Ausbildung des konischen Endbereichs umgekehrt wird;
   - Bilden einer tropfenförmigen Schmelzzone, wobei für ein zweites Zeitintervall die Bewegung des Impfkristalls gestoppt und die Bewegungsrichtung der Siliciumprobe erneut umgekehrt wird;
   - Trennen von Impfkristall und Siliciumprobe, wobei die Bewegungsrichtung der Siliciumprobe umgekehrt wird und diese für eine Dauer von 5 bis 20 s eine Bewegungsgeschwindigkeit von 150 bis 400 mm/min aufweist.

2. Verfahren nach Anspruch 1, **dadurch gekennzeichnet, dass** nach dem Rückschmelzen die Siliciumprobe in einem Endbereich der Länge l einen Durchmesser aufweist, der kleiner oder gleich dem Durchmesser ist, den der Einkristall an seiner Kontaktfläche mit der Schmelze aufweist.

3. Verfahren nach Anspruch 2, **dadurch gekennzeichnet, dass** der Durchmesser der Kontaktfläche des Einkristalls mit der Schmelze 3 bis 8 mm, bevorzugt 4 bis 6 mm, beträgt.

4. Verfahren nach Anspruch 2 oder 3, **dadurch gekennzeichnet, dass** der Durchmesser der Siliciumprobe in ihrem Endbereich der Länge l 2 bis 8 mm, bevorzugt 3 bis 6 mm, beträgt.

5. Verfahren nach einem der Ansprüche 2 bis 4, **dadurch gekennzeichnet, dass** die Länge l des Endbereichs der Siliciumprobe ihrem einfachen bis dreifachen Durchmesser entspricht.

6. Verfahren nach einem der vorhergehenden Ansprüche, **dadurch gekennzeichnet, dass** die Siliciumprobe beim Rückschmelzen mit einer höheren Bewegungsgeschwindigkeit bewegt wird als der Einkristall.

7. Verfahren nach Anspruch 6, **dadurch gekennzeichnet, dass** die Bewegungsgeschwindigkeit der Siliciumprobe 5 bis 15 mm/min, bevorzugt 7 bis 13 mm/min, besonders bevorzugt 9 bis 11 mm/min, beträgt.

8. Verfahren nach Anspruch 6 oder 7, **dadurch gekennzeichnet, dass** die Bewegungsgeschwindigkeit des Einkristalls 2 bis 10 mm/min, bevorzugt 3 bis 8 mm/min, besonders bevorzugt 4 bis 6 mm/min, beträgt.

9. Verfahren nach einem der vorhergehenden Ansprüche, **dadurch gekennzeichnet, dass** das erste Zeitintervall beim Rückschmelzen 30 bis 300 s, bevorzugt 90 bis 240 s, besonders bevorzugt 60 bis 120 s, dauert.

10. Verfahren nach einem der vorhergehenden Ansprüche, **dadurch gekennzeichnet, dass** für das Bilden der tropfenförmigen Schmelzzone die Bewegungsgeschwindigkeit der Siliciumprobe 1 bis 5 mm/min, bevorzugt 2 bis 4 mm/min, beträgt.

11. Verfahren nach einem der vorhergehenden Ansprüche, **dadurch gekennzeichnet, dass** das zweite Zeitintervall 1 bis 4 s, bevorzugt 2 bis 3 s, dauert.

12. Verfahren nach einem der vorhergehenden Ansprüche, **dadurch gekennzeichnet, dass** zum Trennen von Impfkristall und Siliciumprobe die Bewegungsgeschwindigkeit der Siliciumprobe 250 bis 350 mm/min beträgt.

13. Verfahren nach einem der vorhergehenden Ansprüche, **dadurch gekennzeichnet, dass** zum Abkühlen der tropfenförmigen Schmelze die Bewegung der Siliciumprobe gestoppt und der Impfkristall in seiner ursprünglichen Bewegungsrichtung mit einer Bewegungsgeschwindigkeit von 150 bis 400 mm/min, bevorzugt 250 bis 350 mm/min, von der Siliciumprobe entfernt wird.

14. Verfahren nach einem der vorhergehenden Ansprüche, **dadurch gekennzeichnet, dass** der Siliciumtropfen teilweise durch Eintauchen in die Säure für eine Dauer von 3 bis 15 min, bevorzugt 5 bis 10 min, gelöst wird.

15. Verfahren nach einem der vorhergehenden Ansprüche, **dadurch gekennzeichnet, dass** die Säure eine Mischung aus konzentrierter Salpetersäure und Flusssäure im Verhältnis 4:1 bis 3:1, bevorzugt 2:1 bis 1:1, umfasst.

**Claims**

1. Method for determining metallic impurities in silicon, comprising:

    a) provision of a rodlike silicon sample and a rodlike seed crystal in a zone melting apparatus;
    b) zone melting to form a single silicon crystal having a conical end region, with a droplike melt forming at the end of the single silicon crystal in a separation step;
    c) cooling of the droplike melt to form a solidified silicon drop;
    d) partial or complete dissolution of the silicon drop in an acid;
    e) analysis of the solution obtained in step d) by a trace analysis technique,

    **characterized in that** the separation step comprises the following substeps

    - remelting of the silicon sample to reduce its diameter, where for a first time interval the direction of movement of the silicon sample and of the seed crystal is reversed relative to its previous direction of movement, to form the conical end region;
    - formation of a droplike melting zone, where for a second time interval the movement of the seed crystal is halted and the direction of movement of the silicon sample is reversed again;
    - separation of seed crystal and silicon sample, where the direction of movement of the silicon sample is reversed and said sample for a duration of 5 to 20 s has a speed of movement of 150 to 400 mm/min.

2. Method according to Claim 1, **characterized in that** after the remelting, the silicon sample in an end region of length 1 has a diameter which is less than or equal to the diameter of the single crystal at its contact face with the melt.

3. Method according to Claim 2, **characterized in that** the diameter of the contact face of the single crystal with the melt is 3 to 8 mm, preferably 4 to 6 mm.

4. Method according to Claim 2 or 3, **characterized in that** the diameter of the silicon sample in its end region of length 1 is 2 to 8 mm, preferably 3 to 6 mm.

5. Method according to any of Claims 2 to 4, **characterized in that** the length 1 of the end region of the silicon sample corresponds to one to three times its diameter.

6. Method according to any of the preceding claims, **characterized in that** the silicon sample during remelting is moved at a higher speed of movement than the single crystal.

7. Method according to Claim 6, **characterized in that** the speed of movement of the silicon sample is 5 to 15 mm/min, preferably 7 to 13 mm/min, more preferably 9 to 11 mm/min.

8. Method according to Claim 6 or 7, **characterized in that** the speed of movement of the single crystal is 2 to 10 mm/min, preferably 3 to 8 mm/min, more preferably 4 to 6 mm/min.

9. Method according to any of the preceding claims, **characterized in that** the first time interval during remelting lasts 30 to 300 s, preferably 90 to 240 s, more preferably 60 to 120 s.

10. Method according to any of the preceding claims, **characterized in that** for the formation of the droplike melting zone, the speed of movement of the silicon sample is 1 to 5 mm/min, preferably 2 to 4 mm/min.

11. Method according to any of the preceding claims, **characterized in that** the second time interval lasts 1 to 4 s, preferably 2 to 3 s.

12. Method according to any of the preceding claims, **characterized in that** for the separation of seed crystal and silicon sample, the speed of movement of the silicon sample is 250 to 350 mm/min.

13. Method according to any of the preceding claims, **characterized in that** for the cooling of the droplike melt, the movement of the silicon sample is halted and the seed crystal is removed in its original direction of movement with a speed of movement of 150 to 400 mm/min, preferably 250 to 350 mm/min, from the silicon sample.

14. Method according to any of the preceding claims, **characterized in that** the silicon drop is dissolved partially by immersion in the acid for a duration of 3 to 15 min, preferably 5 to 10 min.

15. Method according to any of the preceding claims, **characterized in that** the acid comprises a mixture of concentrated nitric acid and hydrofluoric acid in a ratio of 4:1 to 3:1, preferably 2:1 to 1:1.

**Revendications**

1. Procédé pour la détermination d'impuretés métalliques dans du silicium,
comprenant :

   a) la mise à disposition d'un échantillon de silicium en forme de tige et d'un germe cristallin en forme de tige dans un dispositif de fusion de zone ;
   b) la fusion de zone avec formation d'un monocristal de silicium doté d'une partie terminale conique, une masse fondue en forme de goutte se formant, dans une étape de séparation, à l'extrémité du monocristal de silicium ;
   c) le refroidissement de la masse fondue en forme de goutte avec formation d'une goutte de silicium solidifiée ;
   d) dissolution complète ou partielle de la goutte de silicium dans un acide ;
   e) analyse de la solution obtenue dans l'étape d) au moyen d'une méthode d'analyse de traces,

   **caractérisé en ce que** l'étape de séparation comprend les étapes partielles suivantes

   - refusion de l'échantillon de silicium pour la réduction de son diamètre, dans lequel pour un premier intervalle de temps, la direction de mouvement de l'échantillon de silicium et du germe cristallin est inversée par rapport à leur direction de mouvement précédente pour la formation de la partie terminale conique ;
   - formation d'une zone de masse fondue en forme de goutte, dans laquelle pour un deuxième intervalle de temps, le mouvement du germe cristallin est stoppé et la direction de mouvement de l'échantillon de silicium est à nouveau inversée ;
   - séparation du germe cristallin et de l'échantillon de silicium, la direction de mouvement de l'échantillon de silicium étant inversée et présentant une vitesse de mouvement de 150 à 400 mm/min pendant une durée de 5 à 20 s.

2. Procédé selon la revendication 1, **caractérisé en ce qu'**après la refusion, l'échantillon de silicium présente, dans une partie terminale de longueur l, un diamètre qui est plus petit ou égal au diamètre que présente le monocristal au niveau de sa surface de contact avec la masse fondue.

3. Procédé selon la revendication 2, **caractérisé en ce que** le diamètre de la surface de contact du monocristal avec la masse fondue est de 3 à 8 mm, préférablement de 4 à 6 mm.

4. Procédé selon la revendication 2 ou 3, **caractérisé en ce que** le diamètre de l'échantillon de silicium dans sa partie terminale de longueur l est de 2 à 8 mm, préférablement de 3 à 6 mm.

5. Procédé selon l'une quelconque des revendications 2 à 4, **caractérisé en ce que** la longueur l de la partie terminale de l'échantillon de silicium correspond à une fois à trois fois son diamètre.

**6.** Procédé selon l'une quelconque des revendications précédentes, **caractérisé en ce que** l'échantillon de silicium, lors de la refusion, est déplacé avec une vitesse de mouvement plus élevée que le monocristal.

**7.** Procédé selon la revendication 6, **caractérisé en ce que** la vitesse de mouvement de l'échantillon de silicium est de 5 à 15 mm/min, préférablement de 7 à 13 mm/min, particulièrement préférablement de 9 à 11 mm/min.

**8.** Procédé selon la revendication 6 ou 7, **caractérisé en ce que** la vitesse de mouvement du monocristal est de 2 à 10 mm/min, préférablement de 3 à 8 mm/min, particulièrement préférablement de 4 à 6 mm/min.

**9.** Procédé selon l'une quelconque des revendications précédentes, **caractérisé en ce que** le premier intervalle de temps lors de la refusion dure 30 à 300 s, préférablement 90 à 240 s, particulièrement préférablement de 60 à 120 s.

**10.** Procédé selon l'une quelconque des revendications précédentes, **caractérisé en ce que** pour la formation de la zone de fusion en forme de goutte, la vitesse de mouvement de l'échantillon de silicium est de 1 à 5 mm/min, préférablement de 2 à 4 mm/min.

**11.** Procédé selon l'une quelconque des revendications précédentes, **caractérisé en ce que** le deuxième intervalle de temps dure 1 à 4 s, préférablement 2 à 3 s.

**12.** Procédé selon l'une quelconque des revendications précédentes, **caractérisé en ce que** pour la séparation du germe cristallin et de l'échantillon de silicium, la vitesse de mouvement de l'échantillon de silicium est de 250 à 350 mm/min.

**13.** Procédé selon l'une quelconque des revendications précédentes, **caractérisé en ce que** pour le refroidissement de la masse fondue en forme de goutte, le mouvement de l'échantillon de silicium est stoppé et le germe cristallin est éliminé de l'échantillon de silicium dans sa direction de mouvement d'origine avec une vitesse de mouvement de 150 à 400 mm/min, préférablement de 250 à 350 mm/min.

**14.** Procédé selon l'une quelconque des revendications précédentes, **caractérisé en ce que** la goutte de silicium est dissoute partiellement par immersion dans l'acide pour une durée de 3 à 15 min, préférablement de 5 à 10 min.

**15.** Procédé selon l'une quelconque des revendications précédentes, **caractérisé en ce que** l'acide comprend un mélange d'acide nitrique concentré et d'acide fluorhydrique en un rapport de 4 : 1 à 3 : 1, préférablement de 2 : 1 à 1 : 1.

Fig. 1

Fig. 2

Fig. 3

Fig. 4

Fig. 5

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0349117 A2

- DE 102010039755 A1

**In der Beschreibung aufgeführte Nicht-Patentliteratur**

- **W. ZULEHNER.** Materials Science and Engineering: B. Elsevier, 03. April 2000, 7-15

- **K. GRAFF.** *Metal Impurities in Silicon-Device Fabrication,* ISBN 978-3-642-62965-5